# EUROPEAN PATENT APPLICATION

(11) **EP 2 606 856 A1**
(43) Date of publication of application: **26.06.2013**
(21) Application number: 10856150.7
(22) Date of filing: 19.08.2010
(51) Int. Cl.: A61F 2/38

(54) **ARTIFICIAL KNEE JOINT**

(71) Applicant: Kyocera Medical Corporation, Osaka-shi, Osaka 532-0003 (JP)
(72) Inventor: HASHIDA, Masahiko, Osaka-shi Osaka 532-0003 (JP)
(74) Representative: HOFFMANN EITLE
(86) International application number: PCT/JP2010/063960
(87) International publication number: WO 2012/023193

(57) **Abstract**

An artificial knee joint comprises a femoral component a tibial plate slidably receive the femoral component. The femoral component includes medial and lateral condyles, an opening and an elliptical spherical sliding portion are formed therebetween. The elliptical spherical sliding portion couples posterior ends of the medial and lateral condyles, and slides with respect to the tibial plate when flexing the knee joint. The tibial plate includes a medial fossa and a lateral fossa, and a spine and a concave sliding surface are formed therebetween. The spine moves within the opening in an antero-posterior direction in response to flexion-extension action of the knee joint, and comes into contact with the sliding portion when flexing. The concave sliding surface forms a posterior surface of the spine, and slidably receives the elliptical spherical portion. A width of the elliptical spherical sliding portion is widened from the opening toward a posterior end.

## Description

### Technical Field

The present invention relates to artificial knee joints, and more particularly, to an artificial knee joint whose rotation resistance can be controlled according to a flexion angle. The present artificial knee joint can have high stability in the antero-posterior direction and can also reduce local wear of a post.

### Background Art

When the knee joint is deformed seriously due to a degenerative knee joint disease or a chronic rheumatism, a replacement surgery of an artificial knee joint is performed to restore the normal function of the knee joint.

The artificial knee joint includes a femoral component fixed to a distal end of a femur, and a tibial component fixed to a proximal end of a tibia (see, for example, Patent Documents 1 to 3) . The tibial component includes a tibial tray made of metal, ceramic, or resin directly fixed to the tibia, and a resin tibial plate fixed to an upper surface of the tibial tray in contact with the femoral component.

In flexion of the knee joint, the femoral component rotates while sliding along the surface of the tibial plate. At this time, the femoral component is subjected to the anterior force. When a crucial ligament is cut, the knee joint is possibly dislocated, specifically, the femoral component can be dislocated anteriorly.

From the clinical viewpoint, it is observed that the normal knee joint rotates slightly externally in the extension position and the slight flexion position, but rotates largely externally in the deep flexion position. This is because the crucial ligament has an important role in suppressing the excessive rotation of the knee joint in the extension and slight flexion positions. Thus, when the crucial ligament is cut, the knee joint also possibly becomes unstable in the direction of rotation in the extension and slight flexion positions.

In order to prevent dislocation of the femoral component in the anterior direction in flexion of the knee joint, Patent Document 1 discloses that a cam is provided at the posterior end of the femoral component between a medial condyle and a lateral condyle, while a post protruding superiorly is provided in the center of the tibial component. In flexion of the knee joint, the cam is brought into contact with a posterior surface of the post to prevent the anterior translation of the femoral component.
In order to reduce swinging of the joint in the rotation direction upon the extension and slight flexion, the post interferes with an opening between the femoral condyles in the rotation direction.

In the technique disclosed in Patent Document 1, however, the load from the femoral component concentrates on the post in flexion, so that the post drastically wears locally to be deformed or broken.
Also, in the deep flexion, the post interferes with the opening between the femoral condyles at a small rotation angle, which cannot expect the knee to rotate largely.

In order to solve the problems of the rotation flexibility and wear of a cam-post structure disclosed in Patent Document 1, an artificial knee joint disclosed in Patent Documents 2 and 3 have the following structure. That is, a convex sliding surface is provided at the posterior end of the femoral component and between the medial condyle and the lateral condyle, and a concave sliding surface is provided in the center of a posterior portion of the tibial component. In flexion of the knee joint, the convex sliding surface is brought into contact with the concave sliding surface to prevent the anterior translation of the femoral component. The load of the femoral component in flexion is widely spread over the concave sliding surface, which reduces the local wear of the concave sliding surface.
However, when the tensile force of the ligament cannot be appropriately controlled, the resistance of the knee joint in the antero-posterior direction and in the rotation direction in the extension and slight flexion positions becomes relatively low, which makes the knee joint unstable in the antero-posterior direction and rotation direction.
Patent Document 1: US 4,298,992
Patent Document 2: JP 2981917 B
Patent Document 3: WO 2007/116232

### Disclosure of the Invention

### Problems to be Solved by the Invention

Some of patients having replacement of an artificial knee joint have the ligament (specifically, crucial ligament) removed. In such a case, the artificial knee joint becomes unstable in the antero-posterior direction and in the rotation direction. Specifically, when the tensile force of the ligament of the knee joint cannot be appropriately controlled, the femoral component is not stabilized in the antero-posterior direction and in the rotation direction. The artificial knee joint is desired to be stable in the antero-posterior direction and in the rotation direction in the extension and slight flexion positions. In contrast, the artificial knee joint is desired to have high rotation flexibility in the deep flexion. That is, the artificial knee joint is required to simultaneously achieve these opposite characteristics. Further, the artificial knee joint is also required to ensure abrasion resistance of the post at the same time.

The artificial knee joint disclosed in Patent Document 1 ensures the stability in the antero-posterior direction and in the extension and slight flexion positions, but has a low resistance to abnormal abrasion of the post, and cannot get the high rotation flexibility required in the deep flexion. When the tensile force of the ligament of the knee joint cannot be appropriately controlled, the artificial knee joint disclosed in Patent Documents 2 and 3 cannot be stabilized in the antero-posterior direction and in the rotation direction in the extension and slight flexion positions because of a relatively small resistance in both the atenero-posterior and rotation directions.

An object of the present invention is to provide an artificial knee joint that has high stability in the antero-posterior direction and in the rotation direction even when the tensile force of the ligament of the knee joint cannot be appropriately controlled, and which can achieve the necessary large rotation in the deep flexion. That is, it is an obj ect of the present invention to provide an artificial knee joint whose rotation can be controlled according to an angle of flexion. Another object of the present invention is to provide an artificial knee joint having high resistance to abrasion of a post.

### Means for Solving the Problems

The artificial knee joint of the present invention comprises: a femoral component fixed to a distal end of a femur; and a tibial plate fixed to a proximal end of a tibia and slidably receiving the femoral component, the femoral component including a medial condyle and a lateral condyle, an opening and an elliptical spherical sliding portion being formed between the medial condyle and the lateral condyle, the elliptical spherical sliding portion coupling posterior ends of the medial condyle and the lateral condyle, and sliding with respect to the tibial plate when flexing the knee joint, the tibial plate including a medial fossa for receiving the medial condyle and a lateral fossa for receiving the lateral condyle, a spine and a concave sliding surface being formed between the medial fossa and the lateral fossa, the spine moving within the opening in an antero-posterior direction in response to a flexion- extension action of the knee joint and coming into contact with the elliptical spherical sliding portion when flexing the knee joint, the concave sliding surface forming a posterior surface of the spine and slidably receiving the elliptical spherical portion, a width of the elliptical spherical sliding portion being widened (spread) from the opening toward a posterior end.

As used herein, the phrase "widen from the opening toward a posterior end" means the case in which the width of the elliptical spherical sliding portion is "constantly" or "continuously" widen (spread) from the opening toward the posterior end, and the case in which the width of the sliding portion is partly constant in the midway from the opening to the posterior end, but is widened as a whole. In other words, the phrase "widen from the opening toward the posterior end" means that the width of the elliptical spherical sliding portion does not become narrow in the midway from the opening to the posterior end.
As used herein, the phrase "elliptical spherical sliding portion" means a sliding portion which has a curved surface of an elliptical spherical sliding member as a sliding surface, and can contain all or a part of the elliptical spherical sliding member.
As used herein, the phrase "elliptical spherical member" means not only a three-dimensional body having an elliptical spherical shape with a long axis and a short axis, but also a spherical ball.

### Effects of the Invention

In the artificial knee joint according to the present invention, the width of the elliptical spherical sliding portion is widened from the opening toward the posterior end. When the angle of flexion of the knee joint becomes large, the width of the elliptical spherical sliding portion is increased with respect to the width of the spine. That is, when the angle of flexion of the knee joint becomes large, the flexibility of the elliptical spherical sliding portion in the rotation direction is enhanced. Thus, the artificial knee joint of the present invention can control the restriction of rotation of the knee joint according to the angle of flexion.
In the artificial knee joint of the present invention, the spine comes into contact with the elliptical spherical sliding portion in flexion of the knee joint causing the rotation, which can improve the stability in the rotation direction.

### Brief Description of the Drawings

Fig. 1 is a schematic perspective view of an artificial knee join in extension according to a first embodiment of the invention.
Fig. 2 is a schematic exploded view of the artificial knee joint according to the first embodiment.
Fig. 3 (a) is a schematic cross-sectional view taken along the line α-α line of Fig. 1.
Fig. 3 (b) is a schematic cross-sectional view in flexion of the artificial knee joint shown in Fig. 3 (a) at an angle of 90°.
Figs. 4(a) to 4(e) are schematic cross-sectional views of the artificial knee joint at various angles of flexion in the first embodiment.
Figs. 5(a) to 5(e) are schematic front views of the artificial knee joint at various angles of flexion in the first embodiment.
Figs. 6 (a) to 6 (e) are schematic perspective views of the artificial knee joint at various angles of flexion in the first embodiment.
Figs. 7(a) to 7(c) are schematic perspective views for explaining the external rotation of the artificial knee joint at various angles of flexion in the first embodiment.
Figs. 8(a) to 8F are schematic cross-sectional views of a modified example of the artificial knee joint according to the first embodiment.
Fig. 9 is a schematic perspective view of a distal end of the femur cut.
Figs. 10 (a) to 10 (c) are schematic cross-sectional views of the artificial knee joint at various angles of flexion in the first embodiment.
Figs. 11(a) to 11(c) are schematic perspective views of a conventional knee joint at various angles of flexion in the related art.
Figs. 12 (a) to 12 (c) are schematic perspective views of another conventional knee joint at various angles of flexion in the related art.
Fig. 13(a) is a schematic cross-sectional view of the artificial knee joint in the first embodiment.
Fig. 13 (b) is an exploded view of the artificial knee joint shown in Fig. 13(a).
Figs. 14 (a) to 14 (c) are schematic cross-sectional views of the artificial knee joint at various angles of flexion in the first embodiment.
Fig. 15 is a schematic perspective view of a tibial plate used in an artificial knee joint according to a second embodiment.
Fig. 16 (a) is a schematic cross-sectional view in flexion of the artificial knee joint at an angle of 150° in the second embodiment.
Fig. 16 (b) is a schematic perspective view for explaining the external rotation of an artificial knee joint of a comparative example in flexion at the angle of 150°.
Fig. 17 is an enlarged view of a sliding surface of the posterior end (corresponding to a part I of Fig. 15) of a tibial plate used in the artificial knee joint in the second embodiment.
Fig. 18 is a schematic top view of the tibial plate used in the artificial knee joint in the second embodiment.
Fig. 19 is a schematic top view of the tibial plate used in the artificial knee joint according to the second embodiment.
Fig. 20 is an enlarged view of a posterior portion (corresponding to a part II of Fig. 15) of a medial fossa of the tibial plate used in the artificial knee joint in the second embodiment.
Fig. 21(a) is a schematic cross-sectional view taken along the line Y-Y of Fig. 15.
Fig. 21(b) is a schematic cross-sectional view taken along the line Z-Z of Fig. 15.
Fig. 22 is a schematic cross-sectional view taken along the line Y-Y of Fig. 15.
Figs. 23 (a) and 23 (b) are schematic cross-sectional views of the artificial knee joint in flexion at the angle of 150° in the second embodiment.
Fig. 24 is a schematic perspective view of the tibial plate used in the artificial knee joint in the second embodiment. Description of Reference Numerals

1 Artificial knee joint
10 Tibial plate
11 Medial fossa
11p Cut surface of medial fossa
11r Posterior radius of medial fossa
12 Lateral fossa
12c Posterior end sliding curved surface
12p Posterior end sliding plane
12r Posterior radius of lateral fossa
13 Spine
13t Top of spine
14 Concave sliding surface
20 Femoral component
21 Medial condyle
22 Lateral condyle
23 Opening
24 Elliptical spherical sliding portion
24b Inferior end of elliptical spherical sliding portion

### Mode for Carrying Out the Invention

Embodiments of the present invention will be described in detail below based on the accompanying drawings. In the following description, terms indicative of specific directions and positions (for example, "upper or superior", "lower or inferior", "right", "left", another term including the above terms, and the like) are used if necessary. The use of these terms is for easy understanding of the present invention with reference to the accompanying drawings, and the technical scope of the invention is not limited by the meanings of these terms. Parts represented by the same reference character in a plurality of drawings are the same part or member.

### <First Embodiment>

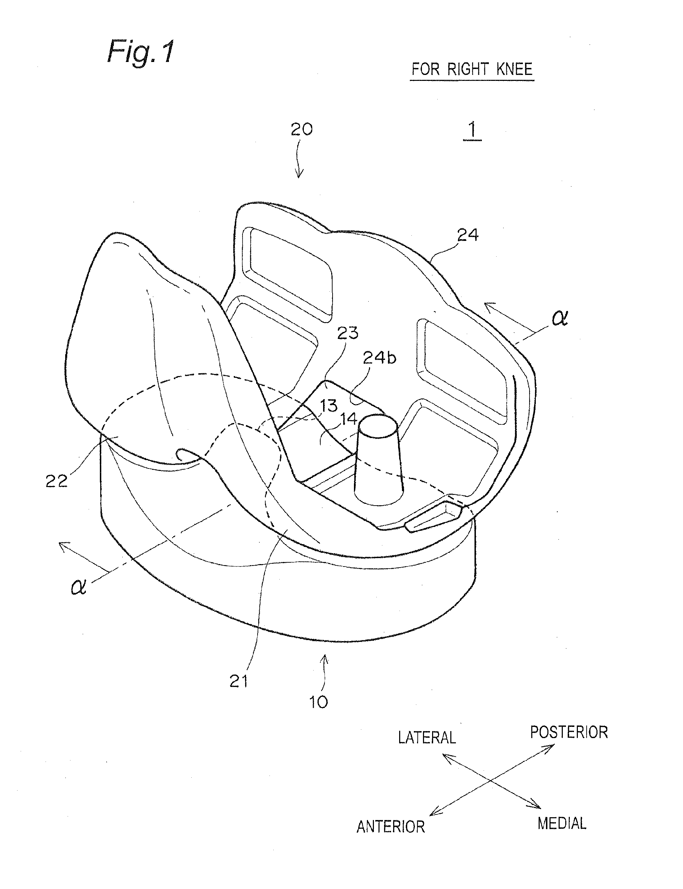

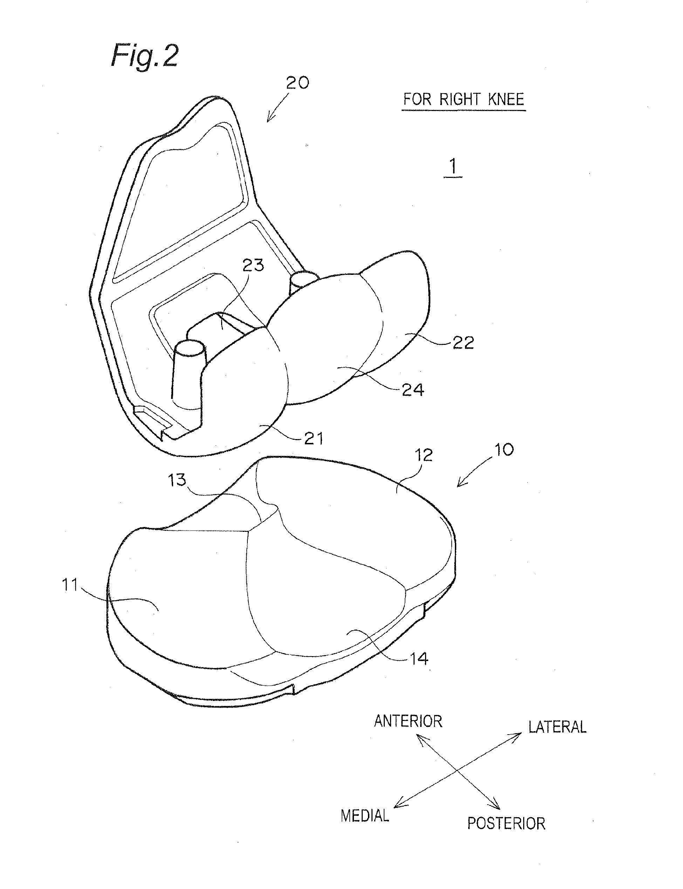

Figs. 1 and 2 show an artificial knee joint 1 in the present embodiment.
The artificial knee joint 1 includes a femoral component 20 fixed to a distal end of a femur, and a tibial plate 10 fixed to a proximal end of a tibia.

The femoral component 20 includes a medial condyle 21 and a lateral condyle 22. Between the medial condyle 21 and the lateral condyle 22 are formed an opening 23 and an elliptical spherical sliding portion 24 for connecting posterior ends of the medial condyle 21 and the lateral condyle 22.

The tibial plate 10 is fixed to the proximal end of the tibia via a metal tibial tray (not shown). The tibial plate 10 includes a medial fossa 11 and a lateral fossa 12. A spine 13 and a concave sliding surface 14 forming the posterior surface of the spine 13 are formed between the medial fossa 11 and the lateral fossa 12.

When the femoral component 20 and the tibial plate 10 form the artificial knee joint 1, the medial condyle 21 of the femoral component 20 is disposed over the medial fossa 11 of the tibial plate 10, and the lateral condyle 22 of the femoral component 20 is disposed over the lateral fossa 12 of the tibial plate 10. The spine 13 of the tibial plate 10 is inserted into the opening 23 of the femoral component 20.

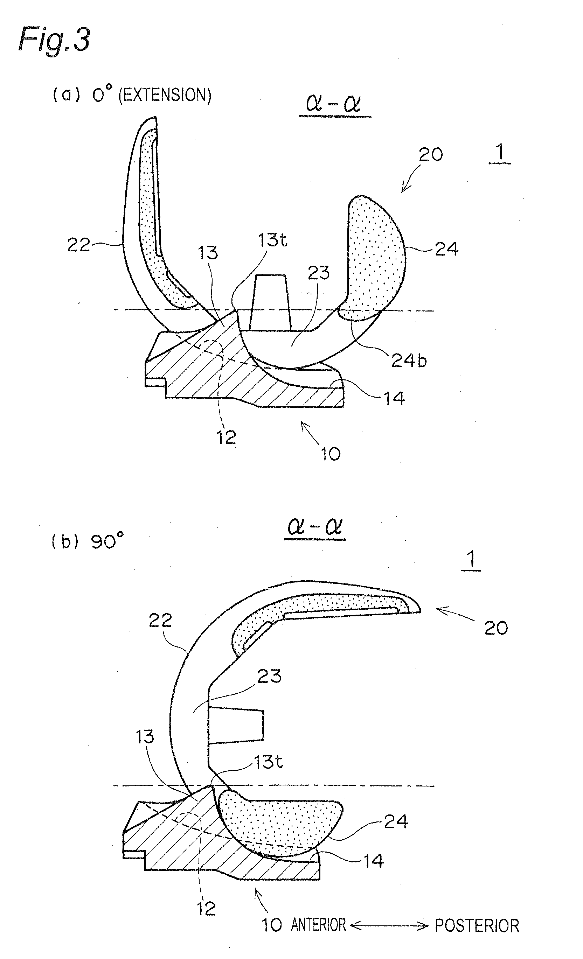

In extension and flexion of the artificial knee joint 1, the medial condyle 21 and the lateral condyle 22 slide in the antero-posterior direction with respect to the medial fossa 11 and the lateral fossa 12. Together with the sliding action, the spine 13 also moves within the opening 23 in the antero-posterior direction (see Figs. 3(a) and 3(b)).

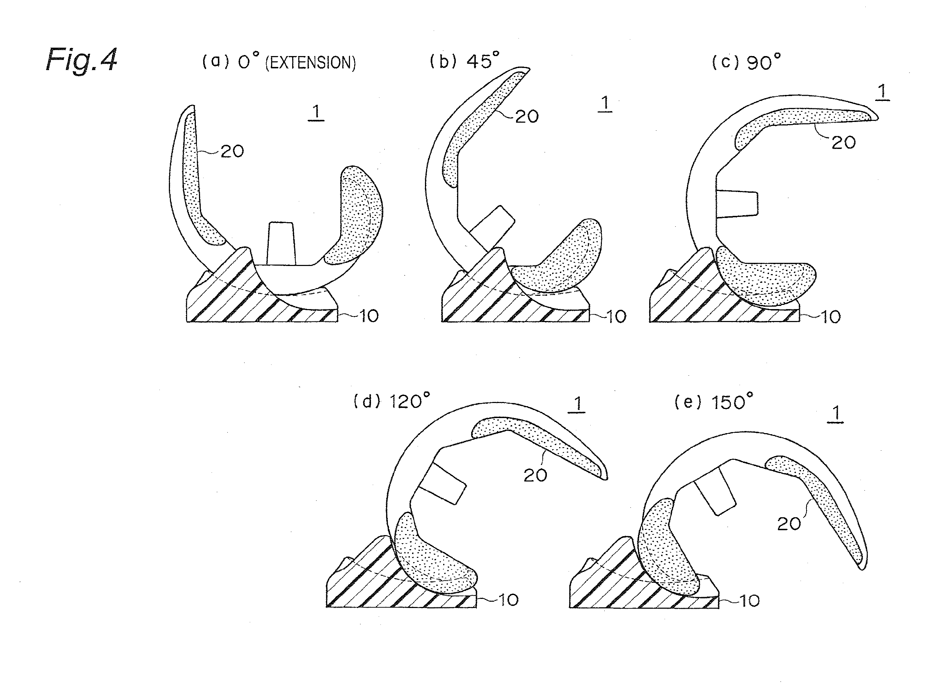

Figs. 4, 5, and 6 show the states of flexion of the artificial knee joint 1 at angles of 0 to 150° in the present embodiment.

### (1) Flexion at Angle of 0° (in Extension) : see Figs. 4 (a), 5 (a), and 6 (a)

In extension of the artificial knee joint 1, the spine 13 is inserted into the opening 23. The elliptical spherical sliding portion 24 is not in contact with the concave sliding surface 14. The medial condyle 21 and lateral condyle 22 of the tibial plate 10 are in contact with the medial fossa 11 and lateral fossa 12 of the femoral component 20, respectively.

### (2) Flexion at Angle of 45°: see Figs. 4(b), 5(b), and 6(b)

The medial condyle 21 and the lateral condyle 22 slides anteriorly with respect to the medial fossa 11 and the lateral fossa 12. Together with the anterior sliding, the spine 13 posteriorly moves within the opening 23. In flexion of the knee joint until an angle of 45°, the elliptical spherical sliding portion 24 formed at the posterior ends of the medial condyle 21 and the lateral condyle 22 is brought into contact with the posterior surface (concave sliding surface 14) of the spine 13. Since the width of the opening 23 is similar to that of the spine 13, the movement of the spine 13 is limited within the opening 23 at an angle in a range of 0 to 15°.

### (3) Flexion at Angle of 90°: see Figs. 4(c), 5(c), and 6(c)

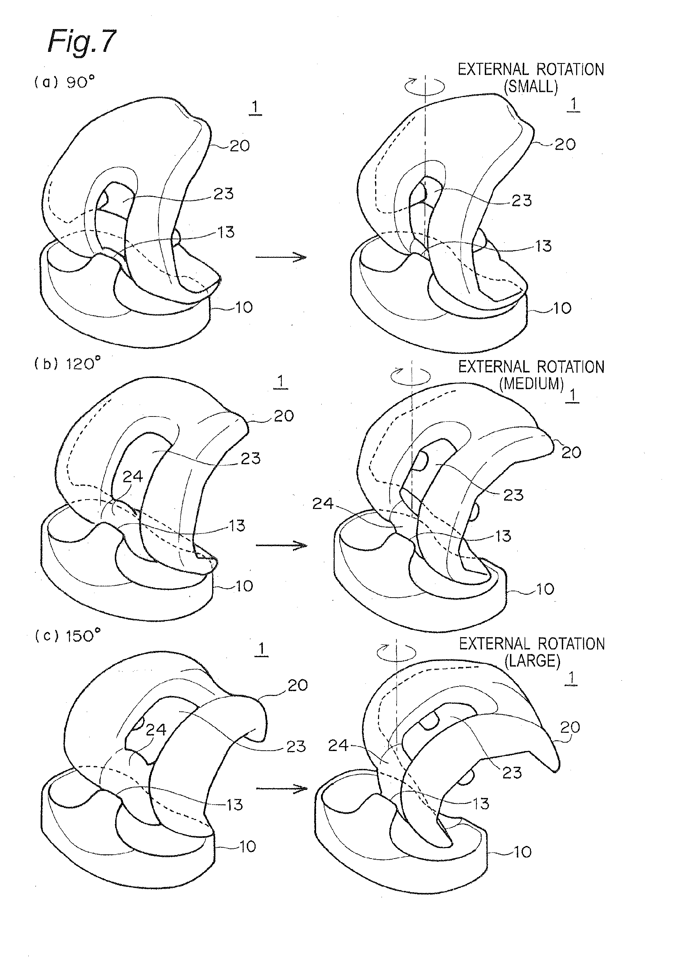

The spine 13 supports the anterior side of the elliptical spherical sliding portion 24 to prevent the dislocation of the femoral component 20 in the anterior direction.
The spine 13 is disengaged from the opening 23. This state releases the restriction of movement of the spine 13 via the opening 23, and then leads to the restriction of rotation of the elliptical spherical portion. Together with this, the femoral component 20 can rotate in a range of 0 to 20° (see Fig. 7(a)).

### (4) Flexion at Angle of 120°: see Figs. 4(d), 5(d), and 6 (d)

The elliptical spherical sliding portion 24 slides against the concave sliding surface 14. The elliptical spherical sliding portion 24 rotates outward, so that the femoral component 20 can externally rotate in a range of 0 to 25° (15) (see Fig. 7(b)).

### (5) Flexion at Angle of 150° : see Figs. 4 (e), 5 (e), and 6(e)

The elliptical spherical sliding portion 24 further slides against the concave sliding surface 14. As a result, the femoral component 20 can externally rotate in a range of 0 to 35° (see Fig. 7(c)).

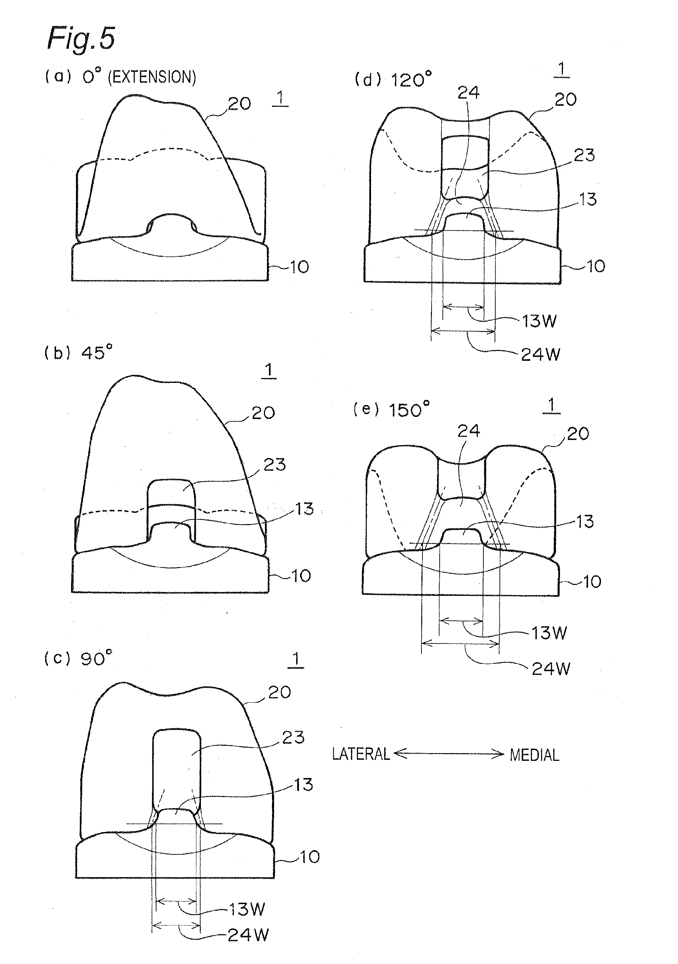

As mentioned above, as the angle of flexion of the artificial knee joint 1 is increased, the femoral component 20 can rotate more externally with respect to the tibial plate 10. The angle that enables external rotation is determined by the relationship between the width 13w of the spine 13 and the width 24w of the elliptical spherical sliding portion 24.
Specifically, the width 24w of the elliptical spherical sliding portion 24 is preferably widened toward the posterior end, which can achieve the same external rotation as that of a natural knee joint (that is, in the slight flexion, the angle for external rotation is small, while in the deep flexion, the angle for external rotation is large) (see Figs. 5(c) to 5(e), and Figs. 7(a) to 7(c)). The relationship between the width 24w of the elliptical spherical sliding portion 24 and the external rotation angle will be described in detail below.

In flexion at an angle of 90° (see Fig. 5(c)), the width 24w of the elliptical spherical sliding portion 24 is slightly wider than the width 13w of the spine 13. Thus, the angle at which the elliptical spherical sliding portion 24 can rotate over the posterior surface (concave sliding surface 14) of the spine 13 is very small (for example, in a range of about 0 to 20°). That is, the femoral component 20 can also externally rotate in an angle range of, for example, 0 to 20° (see Fig. 7(a)).

In flexion at an angle of 120° (see Fig. 5 (d)), the width 24w of the elliptical spherical sliding portion 24 is wider than that of the width 13w of the spine 13. Thus, the range of angle that enables rotation of the elliptical spherical portion 24 becomes wider (for example, in a range of about 0 to 25°). Thus, the femoral component 20 can also externally rotate in an angle range of about 0 to 25° (see Fig. 7(b)).

In flexion at an angle of 150° (see Fig. 5(e)), the width 24w of the elliptical spherical sliding portion 24 is much wider than the width 13w of the spine 13. Thus, the range of angle that enables the rotation of the elliptical spherical sliding portion 24 becomes much wider (for example, in a range of 0 to 35°). The femoral component 20 can also externally rotate in an angle range of, for example, about 0 to 35° (see Fig. 7(b)).

As mentioned above, when the width 24w of the elliptical spherical sliding portion 24 is widened toward the posterior end, the external rotation of the knee joint is restricted in the slight flexion, which can improve the stability of the knee joint. As the range of the angle for external rotation is enlarged with increasing flexion angle, the knee joint can externally rotate at a larger external-rotation angle in deep flexion (for example, at an external-rotation angle of 25 to 35° in flexion at the flexion angle of 135° or more). Thus, the artificial knee joint 1 functioning in the same way as the natural knee joint can be obtained.

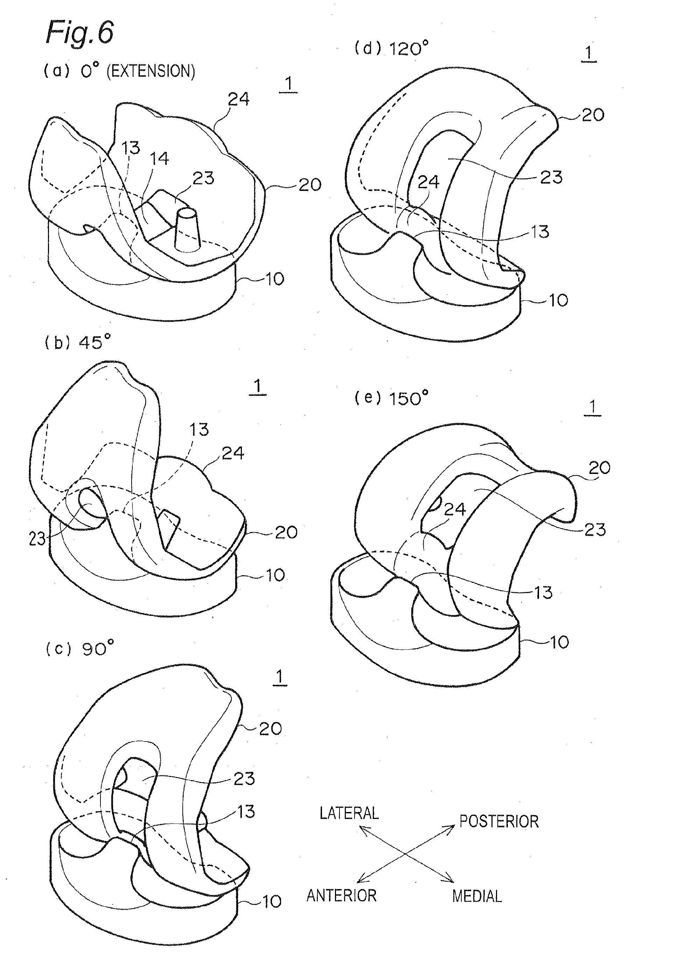

As shown in Figs. 5 and 6, a region between the medial side of the spine 13 and the medial fossa 11 (that is, medial surface of the spine 13), and a region between the lateral side of the spine 13 and the lateral fossa 12 (that is, lateral surface of the spine 13) each preferably have a smoothly curved surface. When the femoral component 20 slides in the antero-posterior direction, and when the femoral component 20 rotates, a contact pressure between the sides of the spine 13 and the medial and lateral condyles 21 and 22 of the femoral component 20 can become low to suppress the abrasion of the spine 13. The edge of the opening 23 (specifically, the edge extending in the antero-posterior direction) of the femoral component 20 more preferably has a curved surface with the substantially same curvature as that of each of the medial and lateral sides of the spine 13.

Turning back to Fig. 3, in the artificial knee joint 1 shown, the elliptical spherical sliding portion 24 is not in contact with the tibial plate 10 in extension of the knee joint 1. In flexion of the artificial knee joint 1 (for example, at an angle of flexion of 90°), the elliptical spherical sliding portion 24 is brought into contact with the concave sliding surface 14 (see Fig. 3(b)). The elliptical spherical sliding portion 24 can slide against the concave sliding surface 14.

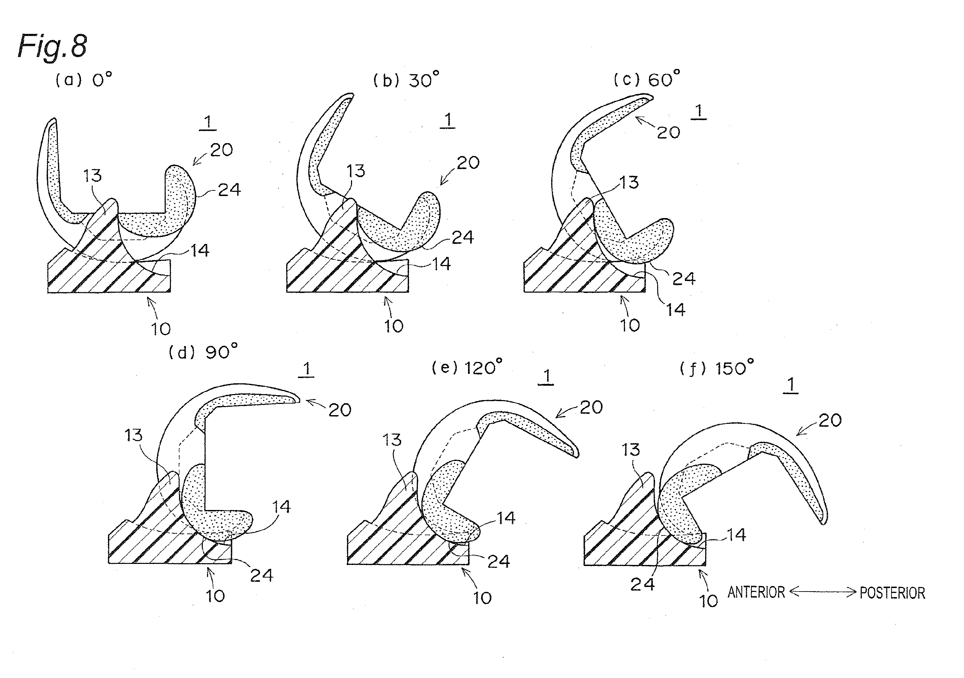

In order to set the angle of flexion to 90° or less to bring the elliptical spherical sliding portion 24 into contact with the concave sliding surface 14, the size and shape of the artificial knee joint can be changed. For example, as shown in Figs. 8(a) to 8(f), the artificial knee joint 1 of the invention includes the artificial knee joint 1 in which the elliptical spherical sliding portion 24 is in contact with the concave sliding surface 14 at an angle of flexion of 0°. In the artificial knee joint shown in the figure, the ellipitical spherical sliding portion 24 is in contact with the tibial plate 10 in all the range of flexion angles of 0 to 150°. The flexion angle at which the elliptical spherical sliding portion 24 comes into contact with the concave sliding surface 14 is preferably in a range of 0 to 90°. If the elliptical spherical sliding portion 24 is not in contact with the concave sliding surface 14 even at an angle of flexion exceeding 90°, the stability of the artificial knee joint 1 is excessively degraded, which is not preferable.

As shown in Fig. 3 (a), in the artificial knee joint 1 of the present embodiment, during the interval from the extension of the knee joint (at an angle of flexion of 0°) to the deep flexion (at an angle of flexion of 135°), a top 13t of the spine 13 is located in a higher position than an inferior end 24b of the elliptical spherical sliding portion 24. (This can be indicated as "JD > 0" using "jumping distance JD" to be described later.) When the femoral component 20 is about to be dislocated anteriorly, the elliptical spherical sliding portion 24 comes into contact with the spine 13. Thus, the femoral component 20 can be prevented from being dislocated anteriorly.

In order to effectively suppress the dislocation of the femoral component 20 in the anterior direction, the top 13t of the spine 13 is more preferably placed in a position higher by about 1 mm or more than the inferior end 24b of the elliptical spherical sliding portion 24 (JD > 1 mm).

In flexion of the artificial knee joint 1, the elliptical spherical sliding portion 24 is in contact with the posterior surface (concave sliding surface 14) of the spine 13 (see Fig. 3(b)). The femoral component 20 is subjected to the anterior force, but the elliptical spherical sliding portion 24 is in contact with the spine 13, so that the femoral component 20 is hardly dislocated anteriorly.

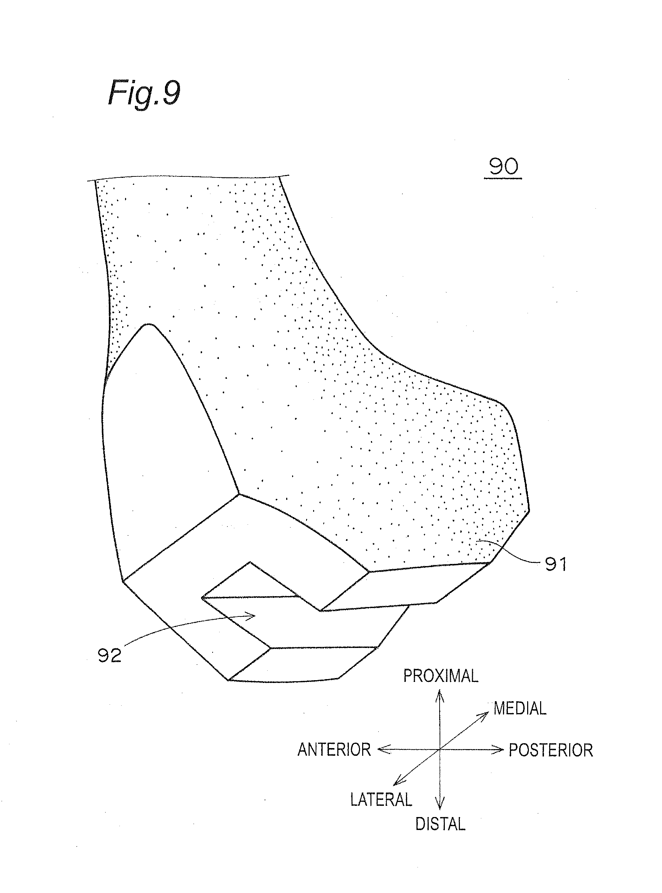

In the artificial knee joint 1 using the tibial plate 10 with the spine 13, the spine 13 is inserted into the opening 23 of the femoral component 20 to protrude into the feral component 20 (or into the region to which the distal end of the femur is fixed). In order not to bring the spine 13 into contact with a femur 90, as shown in Fig. 9, it is necessary to form a space 92 for accommodating therein the spine 13, at a dismal end 91 of the femur 90. The space 92 is formed between the medial condyle and the lateral condyle of the femur 90 (between the condyles) to extend in the antero-posterior direction.

The amount of cutting the bone is preferably reduced so as to keep the strength of the femur 90 high. For this reason, desirably, the amount of protrusion of the spine 13 that protrudes into the femoral component 20 is reduced to narrow the space 92 for accommodating the spine 13 therein. In contrast, when the amount of protrusion of the spine 13 is small, the femoral component 20 tends to be dislocated in the anterior direction. The tendency of dislocation of the femoral component 20 can be recognized from a jumping distance.

The term "jumping distance" as used herein means the "height" of a barrier which the femoral component 20 has to overcome in dislocation in the anterior direction. In the artificial knee joint 1 of the invention, the term "jumping distance" corresponds to a difference in height between the lowest point of the elliptical spherical sliding portion 24 and the top 13t of the spine 13.

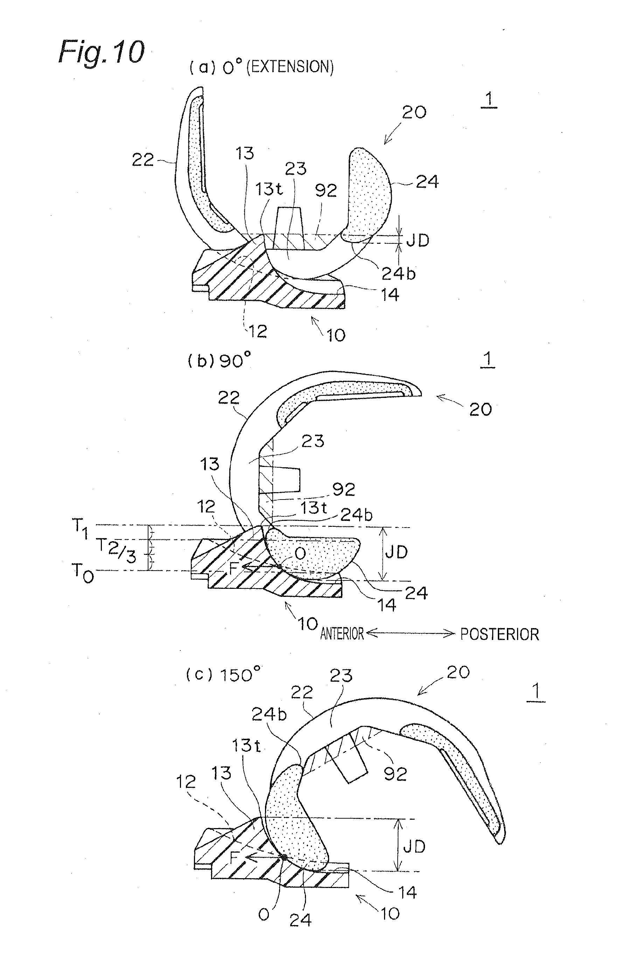

Referring to Fig. 10, a specific example of the jumping distance JD will be described below.
In the artificial knee joint 1 shown in Fig. 10(a), the position of the top 13t of the spine 13 is higher than the position of the lowest point of the elliptical spherical sliding portion 24 (corresponding to the inferior end 24b in the figure) . In this way, when there is the barrier that the femoral component 20 overcomes in dislocation, the jumping distance JD takes a positive value (JD > 0) (hereinafter referred to as a "positive jumping distance"). An absolute value of the jumping distance (hereinafter referred to as a "size of the jumping distance") is equal to the difference in height between the top 13t of the spine 13 and the inferior end 24b of the elliptical spherical sliding portion 24.

As can be seen from Fig. 10 (b), the artificial knee joint 1 at the angle of flexion of 90° has a positive jumping distance JD. The difference between the top 13t of the spine 13 and the lowest point of the elliptical spherical sliding portion 24 is more than the difference shown in Fig. 10(a). The size of the jumping distance JD shown in Fig. 10 (b) is larger than that shown in Fig. 10(a). As the jumping distance JD becomes larger, the femoral component 20 is less likely to be dislocated in the anterior direction. Specifically, in flexion at the angle of 90°, the femoral component 20 is less likely to be dislocated as compared to in flexion at the angle of 0°.
In the artificial knee joint, in the deep flexion, the femoral component 20 is more likely to be dislocated in the anterior direction as compared to in the slight flexion. The artificial knee joint 1 of the invention can effectively suppress the dislocation of the femoral component 20 upon the deep flexion because of the large jumping distance in the deep flexion.

As shown in Fig. 10 (c), the artificial knee joint 1 also has the positive jumping distance at the flexion angle of 150°. The size of the jumping distance of the artificial knee joint 1 shown in Fig. 10(c) is equal to or more than that shown in Fig. 10(b). In the flexion at the angle of 150°, the femoral component 20 is equally or less likely to be dislocated as compared to in the flexion at the angle of 90°.

As can be seen from Fig. 10, in the artificial knee joint 1 of the invention, during the interval from the slight flexion to the deep flexion, the jumping distance becomes positive, which can suppress the dislocation of the femoral component 20 even in the slight flexion and the deep flexion. Since the jumping distance is large in the deep flexion, the dislocation of the femoral component 20 can be effectively suppressed in the deep flexion.

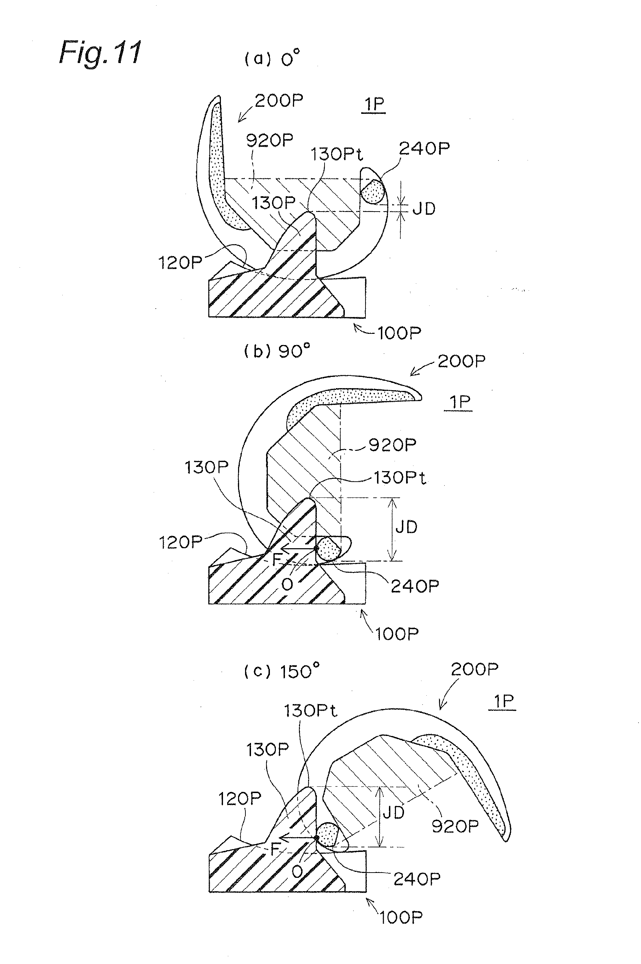

In a conventional artificial knee joint 1P at a flexion angle of 0° as shown in Fig. 11 (see Fig. 11(a)), the lowest point of a cam 240P of a femoral component 200P is located at a higher position than a top 130Pt of a spine 130P (that is, the jumping distance JD is negative (JD < 0)). Thus, the dislocation of the femoral component 200P cannot be suppressed.

At the flexion angles of 90° (see Fig. 11(b)) and 150° (see Fig. 11(c)), the artificial knee joint 1P has the positive jumping distance JD, and thus can suppress the dislocation of the femoral component 200P. The jumping distance JD should be increased so as to effectively suppress the dislocation of the femoral component 200 in the anterior direction. For this reason, the height of the spine 130 needs to be increased. Thus, a space 920P for accommodating therein a spine 130P is widened.

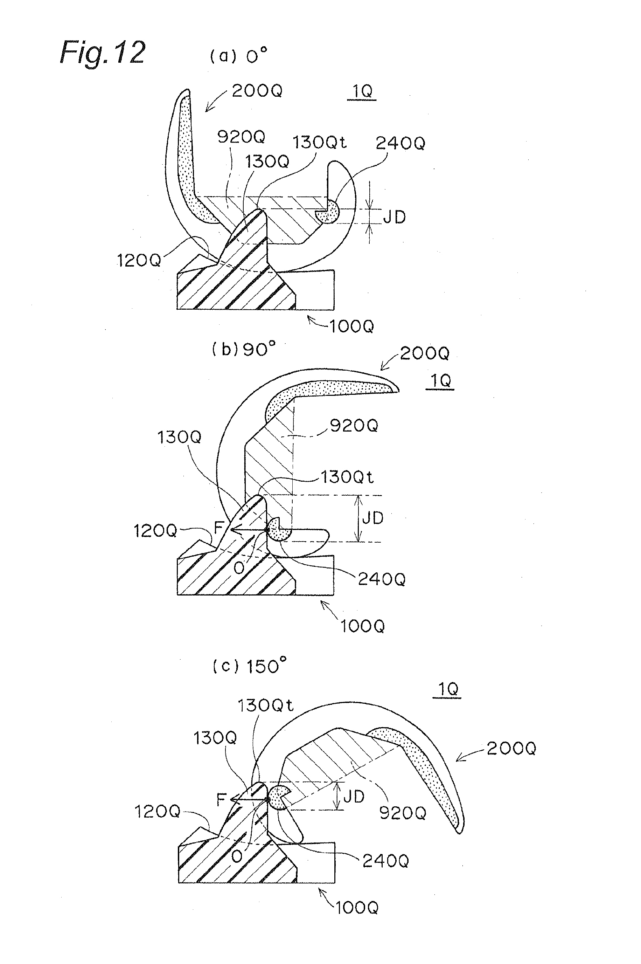

Another conventional artificial knee joint 1Q shown in Fig. 12 has the positive jumping distance JD in all flexions, specifically, at the flexion angle of 0° (see Fig. 12(a)), at the flexion angle of 90° (see Fig. 12(b)), and at the flexion angle of 150° (see Fig. 12(c)).
However, the size of the jumping distance at the flexion angle of 150° is too small. Thus, in the deep flexion, there is the risk that the femoral component 200Q is dislocated.
A point O of action exists near the top of the spine 130Q (which has low strength), so that the spine 130Q is apt to be broken.

In contrast, as shown in Fig. 10, in the artificial knee joint 1 of the invention, the elliptical spherical sliding portion 24 slides into the lower position of the spine 13 (concave sliding surface 14). Even the spine 13 located in the low position can ensure the sufficient jumping distance JD.
Since the spine 13 is low in height, the space 92 for the spine 13 can be made very narrow as compared to that in the related art.

In the artificial knee joint 1 of the invention, the elliptical spherical sliding portion 24 of the femoral component 20 is supported by the posterior surface (concave sliding surface 14) of the spine 13 in flexion, which can ensure the sufficient size of the jumping distance even when the height of the spine 13 is reduced. Thus, the dislocation of the femoral component 20 in the anterior direction can be effectively suppressed, while decreasing the amount of cutting the femur 90.

As can be seen from the comparison between Figs. 10(b) and 10(c), and Figs. 11(b), 11(c), 12(b) and 12(c), the spines 13, 130P, and 130Q receiving the action point O of the femoral components 20, 200P, and 200Q largely differ from each other in thickness. The spine 13 of the present embodiment is twice to three times as thick as that of each of the conventional spines 130P and 130Q. Thus, the spine 13 of the present embodiment is hardly broken even upon being subjected to the large stress F.

In particular, in the flexion position at the flexion angle of 90° or more, the position of the action point O between the elliptical spherical sliding portion 24 and the concave sliding surface 14 is preferably located between the height To of the bottom of the lateral fossa 12 and the height T_{2/3} which is second thirds of a height T₁ of the top 13t of the spine 13 measured from the bottom of the lateral fossa 12. The term "bottom of the lateral fossa 12" as used herein indicates the lowest part of the lateral fossa 12.
The spine 13 has a large thickness between the height To of the bottom and the height T_{2/3}, so that the spine 13 is less likely to be broken even when being subjected to the large stress F from the action point O.

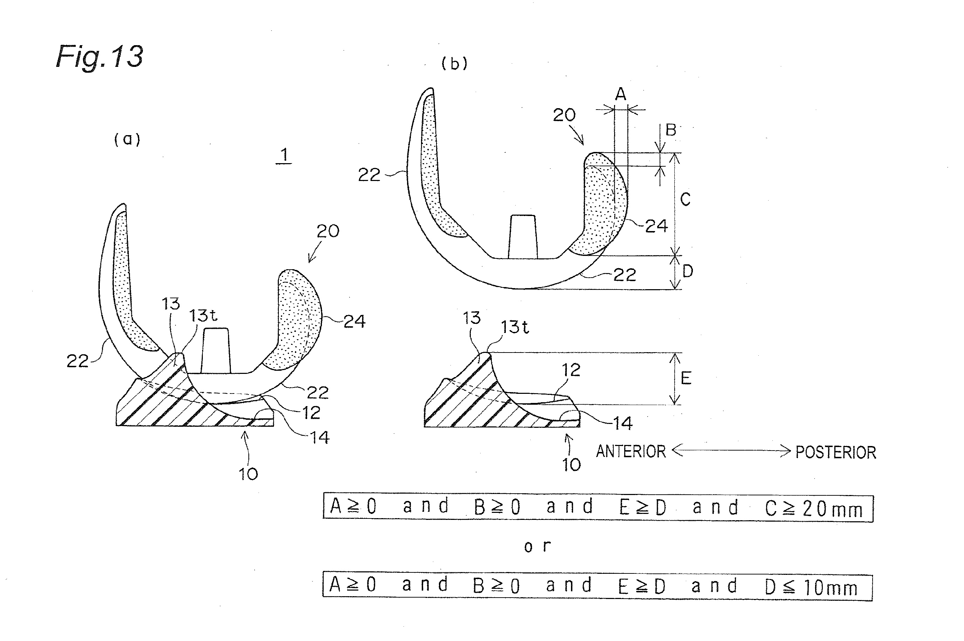

As shown in detail in Fig. 13, at least a part of the elliptical spherical sliding portion 24 of the femoral component 20 desirably protrudes outward from the lateral condyle 22. Referring to Fig. 13, the elliptical spherical sliding portion 24 protrudes from the lateral condyle 22 by a dimension A in the posterior direction and by a dimension (b) at the superior end. When the elliptical spherical sliding portion 24 protrudes outward from the lateral condyle 22 as shown in the figure, the elliptical spherical sliding portion 24 can slide into the lower portion of the spine 13 (concave sliding surface 14), which can further increase the jumping distance JD to lower the position of the action point O.

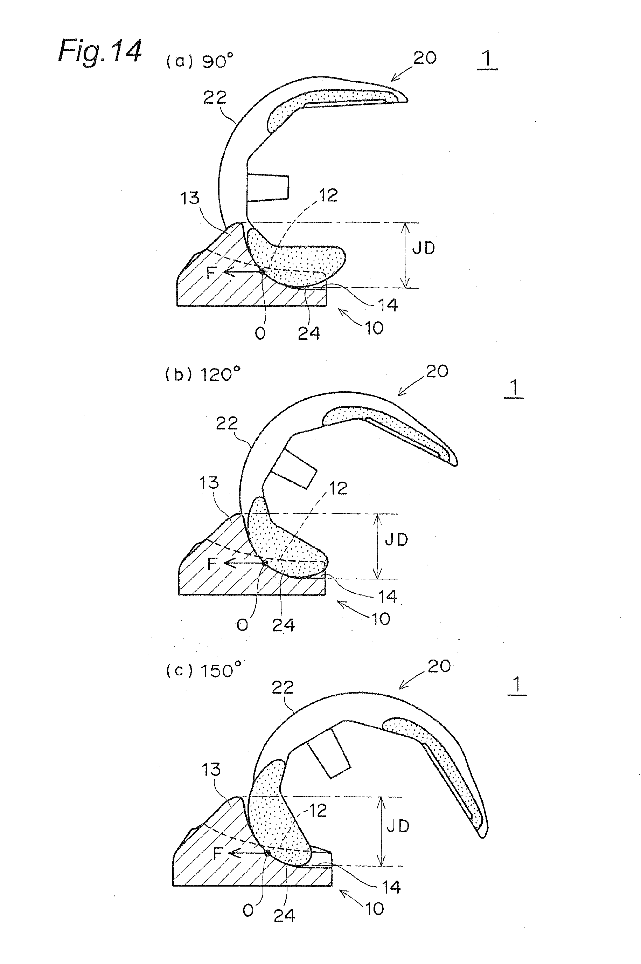

As shown in Fig. 14, even when the flexion angle of the artificial knee joint 1 is 90° or more, the action point O is positioned at the substantially same height as that of the lateral fossa 12, or at the height lower than the lateral fossa 12. Thus, the large jumping distance JD can be ensured, so that the femoral component 20 is less likely to be dislocated in the anterior direction even in the deep flexion. Further, even the repetition of the deep flexion hardly breaks the spine 13 because of a large thickness of the spine 13 supporting the stress F.

### <Second Embodiment>

The artificial knee joint 1 of the present embodiment can externally rotate more easily in flexion. This embodiment differs from the first embodiment in the shape of an edge on the posterior end side of the tibial plate 10. The structures of other components in the present embodiment are the same as those of the first embodiment.

In the present embodiment, in order to promote the external rotation of the knee joint in deep flexion, the tibial plate 10 preferably includes a posterior end sliding surface at the edge of the posterior end side of the lateral fossa 12.

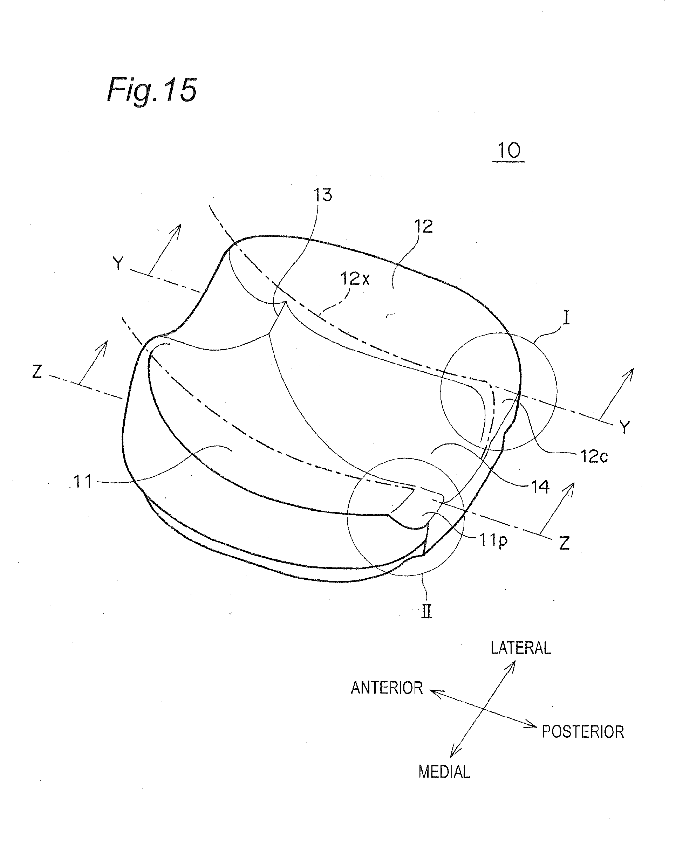

In the present embodiment, as shown in Fig. 15, the posterior end of the lateral fossa 12 of the tibial plate 10 is preferably chamfered by a plane or a curved surface. The chamfered surface forms a posterior end sliding surface (posterior end sliding curved surface 12c, or posterior end sliding plane 12p).
The term "posterior end sliding curved surface 12c" as used in the present specification means all curved posterior end sliding surfaces. As described later, the posterior end sliding surface is a surface along which the lateral condyle 22 of the femoral component 20 slides, as will be described later. Thus, in order to stably slide the lateral condyle 22, the posterior end sliding curved surface 12c is preferably a concave curved surface.
The term "posterior end sliding plane 12p" as used in the present specification means all plane-like posterior end sliding surfaces.

The posterior end sliding surface of the invention will be described below by mainly taking the posterior end sliding curved surface 12c as one example. Unless otherwise specified, in the following, the term "posterior end sliding curved surface 12c" can be replaced by the term "posterior end sliding plane 12p" in reading.

The posterior end sliding curved surface 12c is a surface for causing the lateral condyle 22 of the femoral component 20 to slide, like the lateral fossa 12. The lateral fossa 12 is a surface for causing the lateral condyle 22 to slide before subluxation of the lateral condyle 22. The posterior end sliding curved surface 12c is a surface over which the lateral condyle 22 slides after the subluxation of the lateral condyle 22 (note that Fig. 15 shows a sliding route 12x when the lateral condyle 22 slides over the surfaces of the lateral fossa 12 and posterior end sliding curved surface 12c).

The term "subluxation" as used in the present specification means that the lateral condyle 22 or medial condyle 21 of the femoral component 20 slides posteriorly from the lateral fossa 12 or medial fossa 11 of the tibial plate 10. In the subluxation of the lateral condyle 22, the relative action between the femoral component 20 and the tibial plate 10 becomes similar to the action of the healthy knee joint (the external rotation of the femur). Thus, the tibial plate 10 includes the posterior end sliding curved surface 12c, whereby the tensile force balance of the ligament of the knee can get close to the state of a healthy knee joint, which enables the deep flexion in the same manner as the normal knee joint.

Providing the posterior end sliding curved surface 12c in the tibial plate 10 can form the sliding surface for the knee joint after the subluxation of the lateral condyle 22, and also can promote the subluxation of the lateral condyle 22.
The femoral component 20 is rolled back over the tibial plate 10 by flexion of the knee joint. The deep flexion of the artificial knee joint 1 causes the subluxation of the lateral condyle 22 or medial condyle 21 of the femoral component 20 from the lateral fossa 12 or medial fossa 11 of the tibial plate 10. At this time, when the posterior end sliding curved surface 12c is formed at the posterior end of the lateral fossa 12, the subluxation of the lateral condyle 22 is caused in prior to the medial condyle 21. The artificial knee joint 1 of the present embodiment can promote the subluxation to easily achieve the deep flexion.

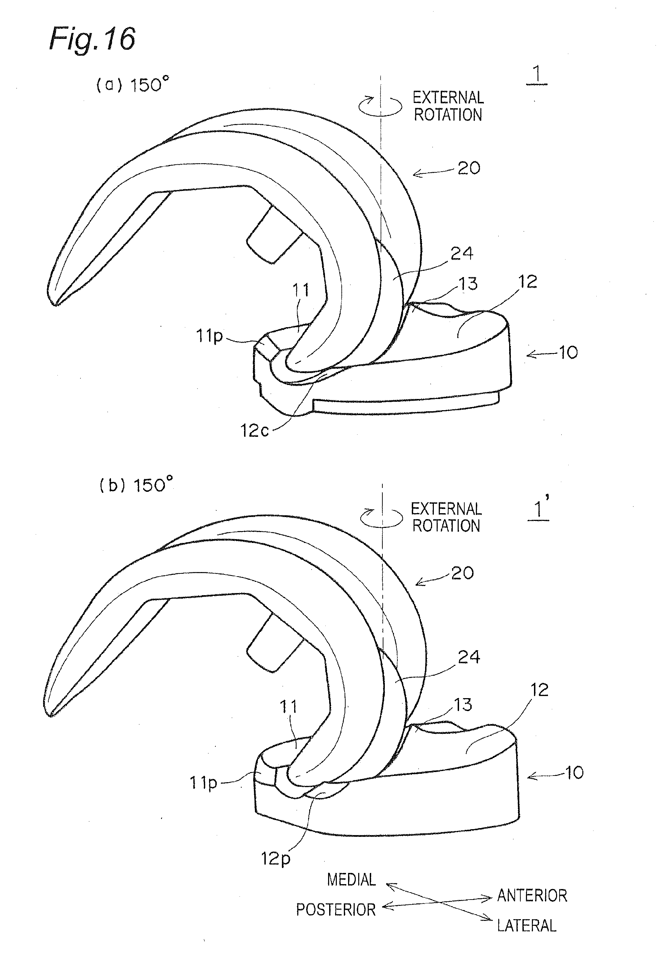

The state of the subluxation of the lateral condyle 22 from the lateral fossa 12 makes the artificial knee joint 1 unstable as compared to the state of non-subluxation of the condyle. In the artificial knee joint 1 of the present embodiment, in the subluxation of the lateral condyle 22, the elliptical spherical sliding portion 24 is brought into contact with the concave sliding surface 14 of the tibial plate 10, which advantageously stabilizes the artificial knee joint 1. After the subluxation of the lateral condyle 22, the femoral component 20 can stably rotate externally with the elliptical spherical sliding portion 24 serving as a supporting point (see Fig. 16 (a)). The knee joint externally rotates with the elliptical spherical sliding portion 24 serving as the supporting point. Thus, the external rotation of the knee joint becomes smooth with the small resistance to the external revolution (as to, for example, the resistance to subluxation or the like).

Fig. 16(a) shows the artificial knee joint 1 with the posterior end sliding curved surface 12c. Fig. 16 (b) shows an artificial knee joint 1' with a posterior end sliding plane 12p not directed in the medial posterior direction, as a comparative example. The artificial knee joint 1 shown in Fig. 16(a) can externally rotate smoothly.

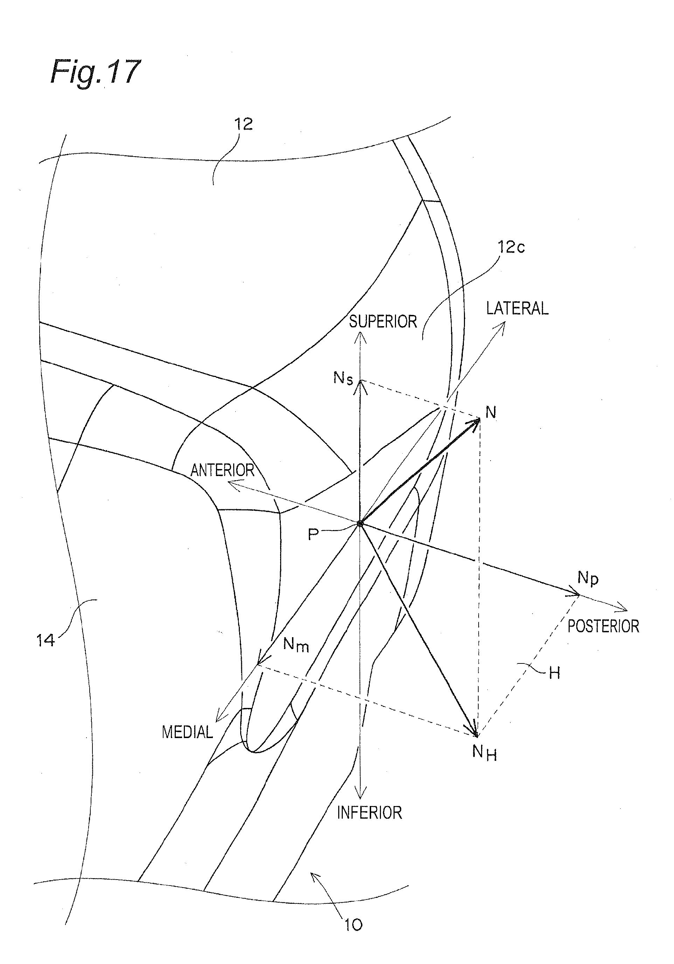

In the present embodiment, the posterior end sliding curved surface 12c is preferably directed in the medial posterior direction.
The term "direction of the posterior end sliding curved surface 12c" will be described in detail below with reference to Fig. 17.

Fig. 17 shows an enlarged view of the posterior end sliding curved surface 12c (part I shown in Fig. 15) of the lateral fossa 12.
Fig. 17 shows the direction of a normal (normal vector N) of the posterior end sliding curved surface 12c at an arbitrary measurement point P. As used herein, the term "direction of the posterior end sliding curved surface 12c" is the direction of the normal vector N. The term "normal vector N" discussed in the present specification is a normal vector including a superior component Ns of two normal vectors that can be drawn respective to the posterior end sliding curved surface 12c.

Referring to Fig. 17, first, the normal vector N is decomposed into the superior component Ns and a horizontal component N_{H} projected onto a horizontal plane H. The horizontal component N_{H} is decomposed into a medial component Nₘ and a posterior component Np. By using these reference characters for the components, the phrase "posterior end sliding curved surface 12c is directed in the medial posterior direction" means that the horizontal component N_{H} of the normal vector N of the posterior end sliding curved surface 12c contains the medial component Nₘ and the posterior component Nₚ.

When the force from the lateral condyle 22 of the femoral component 20 is applied to the posterior end sliding curved surface 12c shown in Fig. 17, the drag is generated in the direction perpendicular to the posterior end sliding curved surface 12c (identical to that of the normal vector N). The drag is applied to the lateral condyle 22 in the medial posterior direction. Thus, the lateral condyle 22 is apt to move in the medial posterior direction. That is, the external rotation of the lateral condyle 22 is promoted.

When the lateral condyle 22 externally rotates in the subluxation, the lateral condyle 22 is supported by the posterior end sliding curved surface 12c, and thus can externally rotate smoothly.

In this way, the posterior end sliding curved surface 12c is directed in the medial posterior direction, which promotes the external rotation of the lateral condyle 22 after the subluxation, and thus externally rotating smoothly.

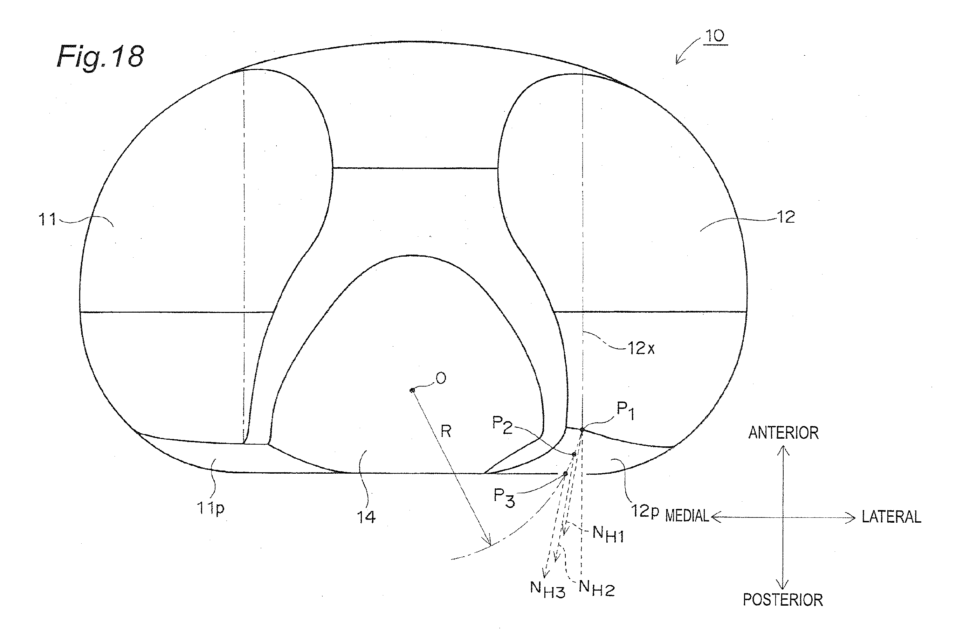

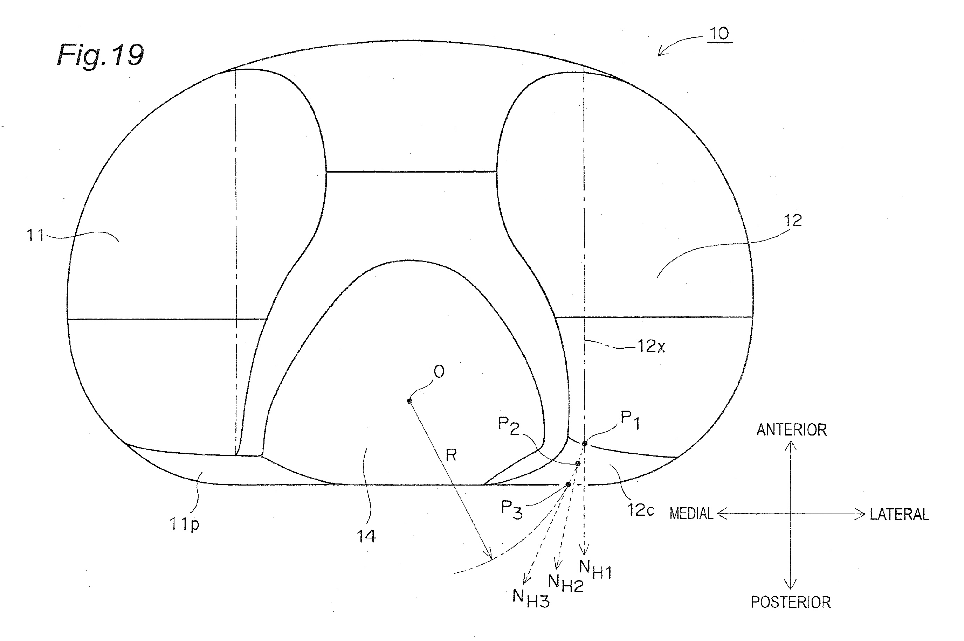

As shown in Figs. 18 and 19, a sliding route 12x of the lateral condyle 22 sliding over the posterior end sliding surface (posterior end sliding curved surface 12c or posterior end sliding plane 12p) can be approximated as a circular arc. As shown in Figs. 18 and 19, the arc of the sliding route 12x is represented by an arc having a radius R with the center O of the concave sliding surface 14 set as the center of the arc.

Figs. 18 and 19 show horizontal components N_{H1} to N_{H3} of the normal vectors N of the posterior end sliding curved surface 12c at any points (P₁ to P₃) on the sliding route 12x. Any one of the horizontal components N₁ to N₃ shown in Figs. 18 and 19 is directed in the medial posterior direction.

Referring to Fig. 18, all three horizontal components N_{H1} to N_{H3} are directed in the same direction. That is, the posterior end sliding surface shown in Fig. 18 is a posterior end sliding plane 12p composed of a flat surface, and directed in the substantially same direction at any points.

In contrast, referring to Fig. 19, three horizontal components N_{H1} to N_{H3} are respectively directed in different directions. That is, the posterior end sliding surface shown in Fig. 19 is the posterior end sliding curved surface 12c comprised of a curved surface. As shown in Fig. 19, preferably, as the point P is positioned posteriorly, the medial component of the horizontal component N_{H} becomes larger. Specifically, by comparison between the horizontal components N_{H1} and N_{H2} at the points P₁ and P₂, the point P₂ is located posteriorly with respect to the point P₁, so that the medial component of the horizontal component N_{H2} is preferably larger than that of the horizontal component N_{H1} (that is, preferably directed more inward).
As the lateral condyle 22 is positioned posteriorly (that is, as the flexion angle of the artificial knee joint 1 becomes larger), the force for pulling the lateral condyle 22 medially is increased, so that together with the force, the angle of external rotation of the lateral condyle 22 can also be increased.
As shown in Fig. 19, the horizontal components N₁ to N₃ coincide with tangential directions of the sliding route 12x at the respective points P₁ to P₃, but are not limited thereto.

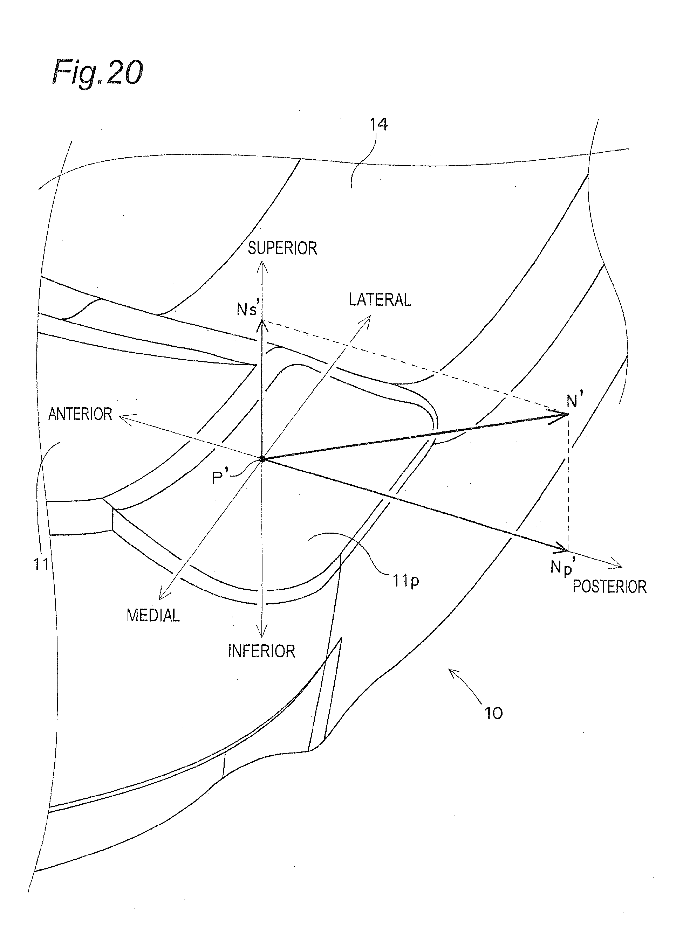

For comparison, the posterior end of the medial fossa 11 of the tibial plate 10 will be described below.
In deep flexion of the artificial knee joint 1, the posterior end of the medial fossa 11 of the tibial plate 10 is brought into contact with the femoral component 20 or femur in some cases. The posterior end of the medial fossa 11 is preferably chamfered by a plane 11p (see Fig. 15). Fig. 20 shows an enlarged view of the plane 11p (part II shown in Fig. 15).
As can be seen from Fig. 20, a normal vector N' drawn from an arbitrary point P' on the plane 11p includes a superior component Nₛ' and a posterior component Nₚ'. However, the normal vector N' does not include the medial component Nₘ'. That is, the plane 11p is directed posteriorly, but not directed in a medial posterior direction.

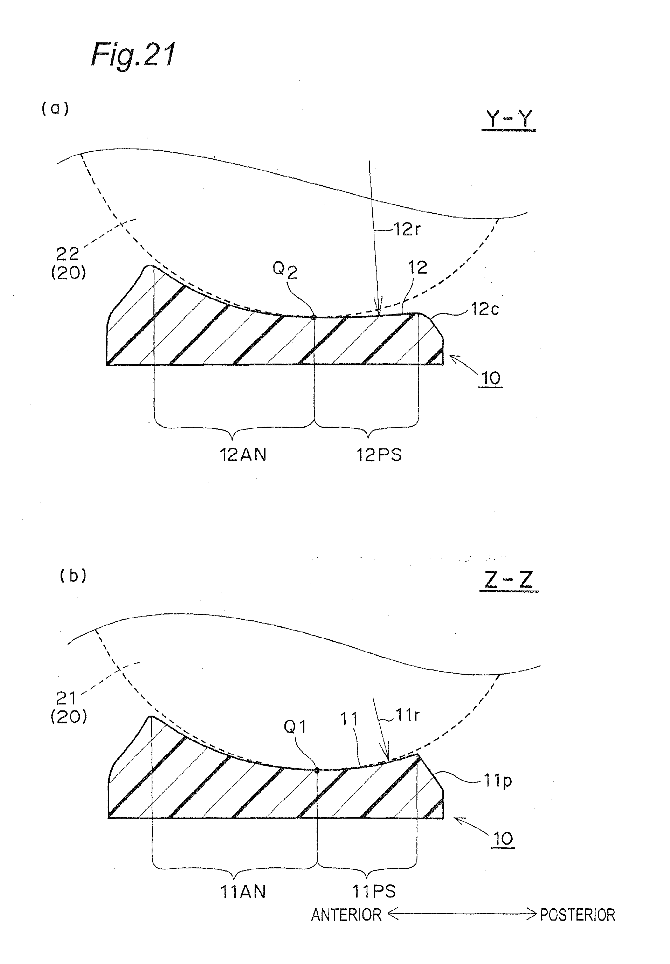

Fig. 21(a) is a diagram of an end surface of the tibial plate 10 in the antero-posterior direction passing through the lowest point (corresponding to a position Q₂) of the lateral fossa 12. Fig. 21 (b) is a diagram of an end surface of the tibial plate 10 in the antero-posterior direction passing through the lowest point of the medial fossa 11. As shown in Figs. 21(a) and 21(b), in the tibial plate 10 used in the artificial knee joint 1 of the invention, the lateral fossa 12 and the medial fossa 11 are curved surfaces.

In the present invention, a radius 12r of a posterior region 12PS of the lateral fossa 12 is preferably larger than a radius 11r of a posterior region 11PS of the medial fossa 11 (see Figs. 21(a) and 21(b)).

As used herein, the term "posterior region 12PS of the lateral fossa 12" is a region of the lateral fossa 12 located posteriorly with respect to the position Q₂ shown in Fig. 21 (a) . The term "posterior region 11PS of the medial fossa 11" is a region of the medial fossa 11 located posteriorly with respect to the position Q₁ shown in Fig. 21(b).
The "position Q₂" of the lateral fossa 12 is a position in which the lowest position of the lateral condyle 22 of the femoral component 20 (indicated by a broken line shown in Fig. 21(a)) is in contact with the lateral fossa 12 of the tibial plate 10 in extension of the artificial knee joint 1. The "position Q₁" of the medial fossa 11 is a position in which the lowest position of the medial condyle 21 of the femoral component 20 (indicated by a broken line shown in Fig. 21(b)) is in contact with the medial fossa 11 of the tibial plate 10 in extension of the artificial knee joint 1.

The term "radius of the posterior region 12PS of the lateral fossa 12" as used in the present specification is a radius of the posterior region 12PS in the section taken along the antero-posterior direction of the lateral fossa 12 (see Fig. 21(a)). Similarly, the term "radius of the posterior region 11PS of the medial fossa 11" is a radius of the posterior region 11PS in the section taken along the antero-posterior direction of the medial fossa 11 (see Fig. 21(b)).

As shown in Figs. 21(a) and 21(b), when the radius 12r of the posterior region 12PS of the outer fossa 12 of the tibial plate 10 is larger than the radius 11r of the posterior region 11PS of the medial fossa 11, the inclination of the posterior region 12PS of the lateral fossa 12 is gentler than that of the posterior region 11PS of the medial fossa 11. That is, when the medial fossa 11 is as high as the lateral fossa 12 in the center of the tibial plate 10 in the antero-posterior direction (for example, in positions Q₁ and Q₂), the lateral fossa 12 is constantly lower than the medial fossa 11 in the respective positions moved posteriorly from the positions Q₁ and Q₂ only by the same distance, respectively. Thus, when the femoral component 20 is rolled back, the lateral condyle 22 is likely to move more posteriorly than the medial condyle 21 in the femoral component 20. As a result, when the rolling back is caused, the lateral condyle 22 tends to be placed more posteriorly than the medial condyle 21, and thus the femoral component 20 is apt to externally rotate. The difference in height between the lateral fossa 12 and the medial fossa 11 of the tibial plate 10 is increased as the points are positioned more posteriorly. The femoral component 20 tends to externally rotate more at the flexion angle (for example, of 135°) for promoting the rolling back than at the flexion angle (for example, of 90°) for starting the rolling back. The use of such a tibial plate 10 can provide the artificial knee joint that can externally rotate more in the deep flexion than in the slight flexion.

The lateral fossa 12 preferably has the radius of the posterior region 12PS that is larger than that of the anterior region 12AN. The medial fossa 11 can have the radius of the posterior region 11PS that is substantially the same as that of the anterior region 11AN. However, the radius of the posterior region 11PS is preferably larger than that of the anterior region 11AN. As used herein, the term "anterior region 12AN of the outer fossa 12" is a region anterior to the position Q₂, and the term "anterior region 11AN of the medial fossa 11" is a region anterior to the position Q₁.

As shown in Fig. 21 (a) , a region between the lateral fossa 12 and the posterior end sliding surface (posterior end sliding curved surface 12c or posterior end sliding plane 12p) is preferably a curved surface, which can reduce the shock on the artificial knee joint at the time of subluxation of the lateral condyle 22 from the lateral fossa 12 toward the posterior end sliding surface.

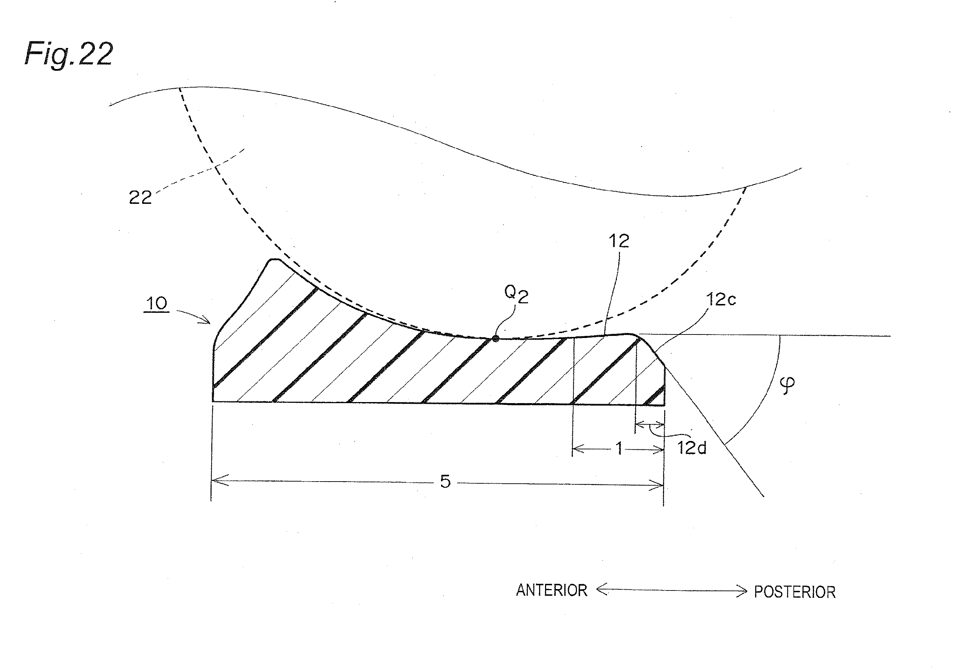

In the section taken along the antero-posterior direction through the lowest point (corresponding to the position Q₂) of the lateral fossa 12 (see Figs. 21(a) and 22), a length 12d in the antero-posterior direction of the posterior end sliding surface (posterior end sliding curved surface 12c or posterior end sliding plane 12p) is preferably one fifth or less of the length of the tibial plate 10 in the antero-posterior direction. Thus, the angle of flexion at which the subluxation of the lateral condyle 22 of the femoral component 20 is caused from the lateral fossa 12 into the posterior end sliding surface can be set within a range of relatively large flexion angles (for example, of 90 to 150°).

In the section taken along the antero-posterior direction through the lowest point of the lateral fossa 12 (see Figs. 21 (a) and 22), an angle of inclination of the posterior end sliding surface (posterior end sliding curved surface 12c or posterior end sliding plane 12p) is preferably 20° or more. The term "angle of inclination of the posterior end sliding surface" as used herein indicates a maximum inclination angle of the posterior end sliding surface (see Figs. 21(a) and 22) in observing the section taken along the antero-posterior direction through the lowest point of the lateral fossa 12.

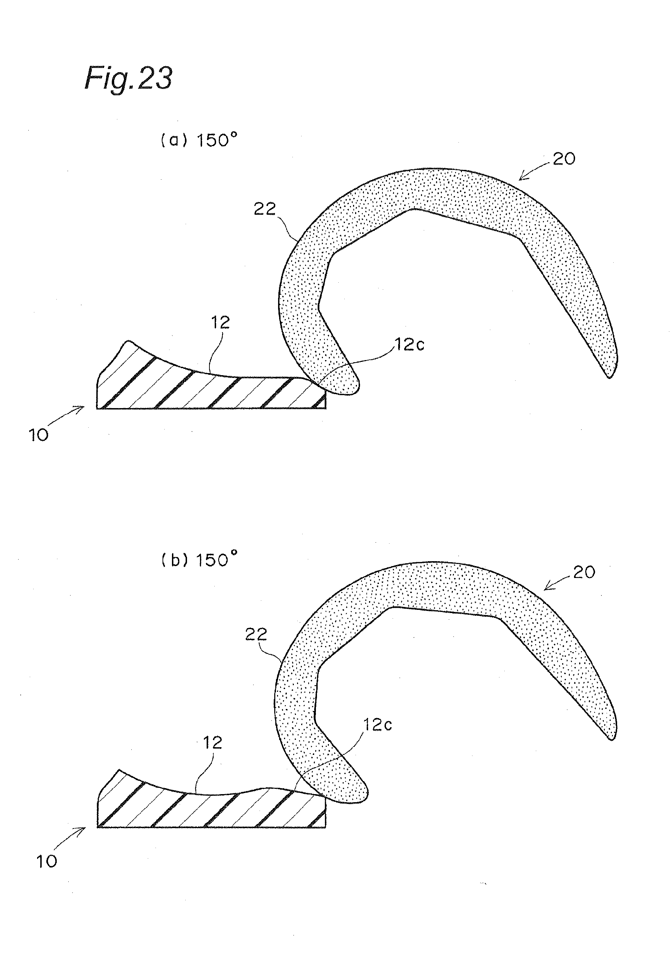

Fig. 23(a) shows the artificial knee joint 1 using the tibial plate 10 in which an angle of inclination of the posterior end sliding surface (posterior end sliding curved surface 12c by way of example) is 20° or more (about 35° in the figure). Fig. 23 (b) shows the artificial knee joint 1 using the tibial plate 10 in which an inclination angle of the posterior end sliding surface is 20° or less (about 10° in the figure).
In flexion of the femoral component 20 at the angle of 150°, as shown in Fig. 23 (a), after the subluxation, the lateral condyle 22 of the femoral component 20 widely comes into contact (surface contact) with the posterior end sliding curved surface 12c of the tibial plate 10. Thus, the lateral condyle 22 can smoothly slide over the posterior end sliding curved surface 12c. In contrast, Fig. 23 (b) shows the lateral condyle 22 in contact with the posterior edge of the posterior end sliding curved surface 12c.

In this way, when the inclination angle of the posterior end sliding curved surface 12c of the tibial plate 10 is 20° or more, the lateral condyle 22 can be received by the surface of the posterior end sliding curved surface 12c even in the deep flexion. As a result, the femoral component 20 can smoothly slide in the deep flexion.

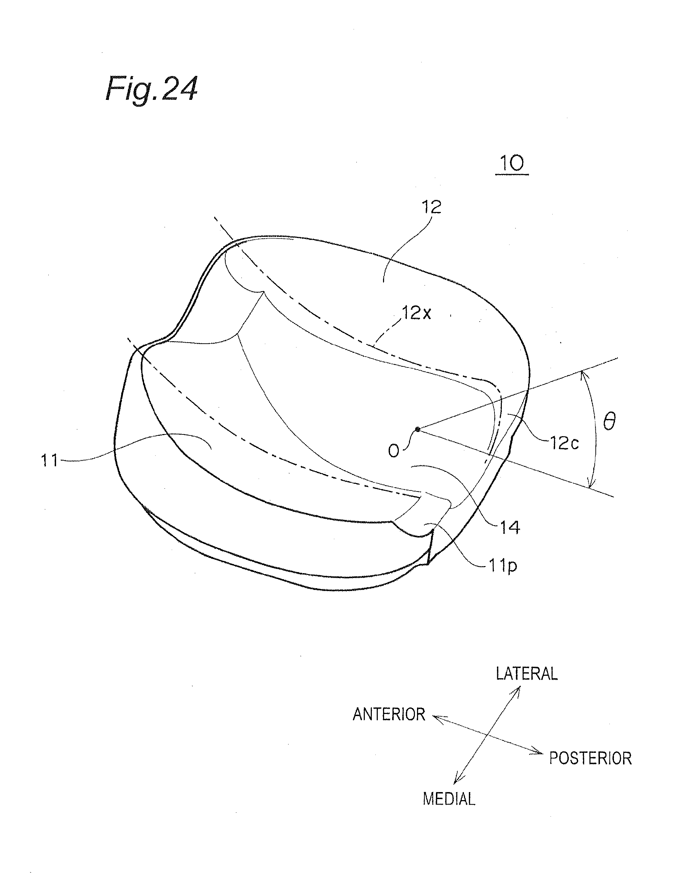

As shown in Fig. 24, the posterior end sliding curved surface 12c is preferably formed in a range of angles 0 of 5 to 30° (θ = 5 to 30°) with the center O of the concave sliding surface 14 set as the center. Thus, the femoral component 20 is apt to externally rotate in a range of external rotation angles (of 5 to 30°) of the natural knee.

The artificial knee joint 1 according to the present embodiment can naturally rotate the femoral component 20 externally in the deep flexion, and thus can achieve the natural action of the knee joint after replacement of the artificial knee joint 1.

## Claims

1. An artificial knee joint, comprising: a femoral component fixed to a distal end of a femur; and a tibial plate fixed to a proximal end of a tibia and slidably receiving the femoral component,
the femoral component including a medial condyle and a lateral condyle,
an opening and an elliptical spherical sliding portion being formed between the medial condyle and the lateral condyle,
the elliptical spherical sliding portion coupling posterior ends of the medial condyle and the lateral condyle, and sliding with respect to the tibial plate when flexing the knee joint,
the tibial plate including a medial fossa for receiving the medial condyle and a lateral fossa for receiving the lateral condyle,
a spine and a concave sliding surface being formed between the medial fossa and the lateral fossa ,
the spine moving within the opening in an antero-posterior direction in response to a flexion-extension action of the knee joint and coming into contact with the elliptical spherical sliding portion when flexing the knee joint,
the concave sliding surface forming a posterior surface of the spine and slidably receiving the elliptical spherical portion,
a width of the elliptical spherical sliding portion being widened from the opening toward a posterior end.

2. The artificial knee joint according to claim 1, wherein in a range of angles of flexion of 0 to 150°, a top of the spine is located in a higher position than an inferior end of the elliptical spherical sliding portion.

3. The artificial knee joint according to claim 1 or 2, wherein surfaces between a medial side of the spine and the medial fossa and between a lateral side of the spine and the lateral fossa are curved surfaces.

4. The artificial knee joint according to any one of claims 1 to 3, wherein a posterior end of the lateral fossa is chamfered by a plane or curved surface to form a posterior end sliding surface, and
wherein the posterior end sliding surface is directed in a medial-posterior direction.

5. The artificial knee joint according to any one of claims 1 to 4, wherein in a flexion position at an angle of flexion of 90° or more, a position of an action point in a height direction between the elliptical spherical sliding portion and the concave sliding surface is positioned between a height of a bottom of the lateral fossa, and a height of a position located at a second thirds of a height of the top of the spine measured from the bottom of the lateral fossa.

6. The artificial knee joint according to any one of claims 1 to 5, wherein at least a part of the elliptical spherical sliding portion protrudes outward from the lateral condyle.
